(19) Europäisches Patentamt

European Patent Office

Office européen des brevets

(11) Veröffentlichungsnummer: **0 214 477**
**A1**

(12) **EUROPÄISCHE PATENTANMELDUNG**

(21) Anmeldenummer: **86110949.4**

(22) Anmeldetag: **08.08.86**

(51) Int. Cl.⁴: **A01N 25/00** , A01N 53/00

(30) Priorität: **20.08.85 DE 3529693**

(43) Veröffentlichungstag der Anmeldung:
**18.03.87 Patentblatt 87/12**

(84) Benannte Vertragsstaaten:
**BE DE FR GB IT NL**

(71) Anmelder: **BAYER AG**
**Konzernverwaltung RP Patentabteilung**
**D-5090 Leverkusen 1 Bayerwerk(DE)**

(72) Erfinder: **Stendel, Wilhelm, Dr.**
**In den Birken 55**
**D-5600 Wuppertal 1(DE)**
Erfinder: **Dorn, Hubert, Dr.**
**Pahlkestrasse 71**
**D-5600 Wuppertal 1(DE)**
Erfinder: **Voege, Herbert, Dr.**
**Martin-Buber-Strasse 41**
**D-5090 Leverkusen 3(DE)**

(54) **Verfahren zur Bekämpfung von Ektoparasiten bei Herdentieren und bei vergesellschaftet lebenden Tieren.**

㊗ Die vorliegende Erfindung betrifft ein Verfahren zur Bekämpfung von Ektoparasiten bei Herdentieren bei dem nur wenige Tiere pro Herde im Aufgußverfahren behandelt werden und die übrigen Tiere unbehandelt bleiben.

EP 0 214 477 A1

# Verfahren zur Bekämpfung von Ektoparasiten bei Herdentieren und bei vergesellschaftet lebenden Tieren

Die vorliegende Erfindung betrifft ein Verfahren zur Bekämpfung von Ektoparasiten bei Herdentieren und bei vergesellschaftet lebeden Tieren.

Es ist bereits bekannt geworden, daß Hornfliegen (Lyperosia irritans) in Rinderherden dadurch bekämpft werden können, daß man nicht alle, sondern nur wenige Tiere pro Herde mit einer Lösung von Permethrin einsprüht.

Es ist außerdem bekannt geworden, Hornfliegen bei Rinderherden dadurch zu bekämpfen, daß man Detektortiere wie Suchbullen mit einem Gerät versieht, aus dem beim Aufspringen der Detektortiere auf andere Tiere eine geringe Menge einer permethrinhaltigen Lösung austritt. Auf diese Weise sorgt ein Tier für die Behandlung mehrerer, vor allem brünstiger Tiere. (JOURNAL Econ. Entomol. 77 (1984) S. 655-656). Der Nachteil dieser Behandlungsmethode ist jedoch, daß das verwendete Gerät gewartet und nachgefüllt werden muß.

Es wurde nun gefunden, daß die Behandlung von Herdentieren und Tieren, die vergesellschaftet leben, zur Bekämpfung von Ektoparasiten dadurch erfolgen kann, daß man pro Herde ein oder mehrere Tiere im Aufgußverfahren mit einem Mittel behandelt, das einen oder mehrere Wirkstoffe aus der Gruppe 2,2-Dimethyl-3-(2,2-dichlor vinyl)-cyclopropan-1-carbonsäure-α-cyano-(3-phenoxy-4-fluor)-benzylester (Cyfluthrin), 2,2-Dimethyl-3-[2-(4-chlorphenyl)-2-chlorvinyl]-cyclopropan-1-carbonsäure-α-cyano-(3-phenoxy-4-fluor)-benzylester (Flumethrin), 2,2-(Dimethyl-3-(2,2-dichlorvinyl)-cyclopropan-1-carbonsäure-α-cyano-3-phenoxy-benzylester (Cypermethrin), 2,2-Dimethyl-3-(2,2-dibromvinyl)-cyclopropan-1-carbonsäure-α-cyano-3-phenoxy-benzylester (Deltamethrin), 2,2-Dimethyl-3-(2,2-dichlorvinyl)-cyclopropan-1-carbonsäure-pentafluorbenzylester (Fenfluthrin), deren Enantiomere oder Diastereomere oder Gemische derselben neben hautverträglichen Verdünnungsmitteln sowie gegebenenfalls Emulgatoren und weiteren Hilfsstoffen enthält.

Überraschenderweise zeigt sich, daß die Behandlung eines oder nur weniger Tiere pro Herde im Aufgußverfahren ausreicht, um bei der gesamten Herde Ektoparasiten zu bekämpfen.

Herdentiere oder vergesellschaftet lebende Tiere, bei denen die Behandlung angewendet werden kann, sind Rinder, Pferde, Schafe, Ziegen aber auch Schweine, Kaninchen und Geflügel, sowie Wildtiere wie Gazellen, Hirsche, Rehe, Rentiere usw. und sog. Labortiere wie Ratten, Mäuse, Meerschweinchen, Hamster usw.

Pro Herde werden ca. 1-25 % der Tiere im Aufgußverfahren behandelt. Bevorzugt werden ca. 10-20 % der Tiere einer Herde behandelt.

Besonders bevorzugt sind Mittel, die Flumethrin und/oder Cyfluthrin und/oder Fenfluthrin allein oder in Mischung miteinander enthalten.

Die Mittel enthalten den Wirkstoff in Konzentrationen von 0,01-10 % bevorzugt von ca. 1 %.

In Abhängigkeit von der Größe der Tiere werden pro Tier 0,1-100 ml, bevorzugt 10-50 ml Mittel angewendet.

So werden bei Rindern 10-100 ml, bevorzugt 20-50 ml, angewendet.

Bei Schafen werden 2-100 ml, bevorzugt 5-20 ml, angewendet.

Bei kleineren Tieren setzt man 1-20, bevorzugt 2-5 ml pro Tier ein.

Die Mittel werden je nach Parasitenart, Parasitendruck und Klima in einem Abstand von 1 Woche bis 6 Monaten, bevorzugt im Abstand von 1 Monat bis 3 Monaten angewendet. Es ist dabei jedoch nicht erforderlich, daß bei jeder Behandlung immer dieselben Tiere wieder behandelt werden.

Das erfindungsgemäße Verfahren läßt sich gegen alle oder einzelne Entwicklungsstadien von Ektoparasiten bei Herdentieren einsetzen. Zu den Ektoparasiten zählen:

Aus dem Stamm der Insekten und der Ordnung Diptera mit den Familen

| Tabanidae | z.B. Tabanus spec. |
|---|---|
| Simulidae | z.B. Simulium spec. |
| Oestridae | z.B. Gastrophilus spec. |
| | Oestrus spec. |
| | Hypoderma spec. |
| | Dermatobia spec. |
| Anthomyidae | z.B. Musca spec. |
| | Stomoxys spec. |
| | Lyperosia spec. |
| | Glossina spec. |
| Tachinidae | z.B. Lucilia spec. |
| | Calliphora spec. |
| | Chrysomyia spec. |
| | Callitroga spec. |
| Hippobiscidae | z.B. Melophagus spec. |

der Ordnung Phthiraptera mit den Familien

Haematopinidae z.B. Haematopimus spec.

Linognathidae z.B. Linognathus spec.

Damalinidae z.B. Damalinea spec.

der Ordnung Siphonaptera mit den Familien

Ctenocephalidae z.B. Ctenocephalides spec.
    Aus dem Stamm der Arachnida und der Ordnung Acarina mit den Familien

| Dermanyssidae | z.B. | Dermanyssus spec. |
| Argasidae | z.B. | Argas spec. |
| | z.B. | Ornithodorus spec. |
| | z.B. | Otobius spec. |
| Ixodidae | z.B. | Ixodes spec. |
| | z.B. | Boophilus spec. |
| | z.B. | Hyalomma spec. |
| | z.B. | Rhipicephalus spec. |
| | z.B. | Haemaphysalis spec. |
| | z.B. | Dermacentor spec. |
| | z.B. | Amblyomma spec. |
| Demodicidae | z.B. | Demodex spec. |
| Cheyletidae | z.B. | Psorergates spec. |
| Myobiidae | z.B. | Myobia spec. |
| Sarcoptidae | z.B. | Sarcoptes spec. |
| | z.B. | Notoedres spec. |
| Psoroptidae | z.B. | Psoroptes spec. |
| | z.B. | Chorioptes spec. |
| | z.B. | Otodectes spec. |

Die Mittel werden in an sich bekannter Weise hergestellt indem der Wirkstoff oder Mischungen der Wirkstoffe in einem oder mehreren hautverträglichen Verdünnungsmitteln gelöst oder suspendiert und gegebenenfalls weitere Hilfsstoffe zugesetzt werden.

Geeignete Verdünnungsmittel sind Alkohole, wie Ethylalkohol, Isopropylalkohole, n-Butylalkohol, Amylalkohol, Octanol.

Glykole, wie Propylenglykol, 1,3-Butylenglykol, Ethylglykol, Dipropylenglykolmonomethylether.

Aromatische Alkohole wie Benzylalkohol.

Carbonsäureester, wie z.B. Ethylacetat, Benzylbenzoat, Butylacetat, Propylencarbonat, Milchsäureethylester.

Aliphatische Kohlenwasserstoffe, Öle, die nicht unter die Definition der spreitenden Öle fallen, wie z.B. Baumwollsaatöl, Erdnußöl, Maiskernöl, Olivenöl, Ricinusöl, Sesamöl.

Ketone, wie z.B. Aceton und Methylethylketon.

Synth. Mono-und Triglyceride mit natürlichen Fettsäuren.

Weiterhin sind u.a. Verbindungen wie Dimethylsulfoxid, Dimethylacetamid, Dimethylformamid, N-Methylpyrrolidon, Dioxan, 2,2-Dimethyl-4-oxymethyl-1,3-dioxolan gut als Verdünnungsmittel geeignet.

Besonders geeignet sind niedere Alkohole mit bis zu 8 Kohlenstoffatomen im Molekül, sowie niedere Ketone wie Methylethylketon und Ether des Ethylenglykols und des Propylenglykols.

Es können bei der Herstellung der erfindungsgemäß verwendbaren Mittel ein oder mehrere Verdünnungsmittel eingesetzt werden.

Als weitere Hilfsstoffe sind geeignet:

a) Haftvermittler, z.B. Carboxymethylcellulose, Methylcellulose und andere Cellulose-und Stärke-Derivate, Polyacrylate, Alginate, Gelatine, Gummi arabicum, Polyvinylpyrrolidon, Polyvinylalkohol, Copolymere aus Methylvinylether und Maleinsäureanhydrid, Polyethylenglykole, Paraffine, Öle, Wachse, hydriertes Rizinusöl.

Kolloidale Kieselsäure oder Gemische der aufgeführten Stoffe. Lösungen und Emulsionen können neben dem Wirkstoff die üblichen Trägerstoffe enthalten, wie

b) Tenside (beinhaltet Emulgatoren und Netzmittel), z.B.

1. anionaktive Tenside, wie Na-Laurylsulfat, Fettalkoholethersulfate, Mono/Dialkylpolyglykoletherorthophosphorsäureester-Monoethanolaminsalz, Calciumalkylarylsulfonat,

2. kationaktive Tenside, wie Cetyltrimethylammoniumchlorid,

3. ampholytische Tenside, wie Di-Na-N-lauryl-beta-iminodipropionat oder Lecithin,

4. nicht ionogene Tenside, z.B. polyoxyethyliertes Ricinusöl, polyoxyethyliertes Sorbitan-Monooleat, Sorbitan-Monostearat, Glycerinmonostearat, Polyoxyethylenstearat, Alkylphenolpolyglykolether.

c) Spreitmittel wie z.B.

Silikonöle verschiedener Viskosität

Fettsäureester wie Ethylstearat, Di-n-butyl-adipat, Laurinsäurehexylester, Dipropylen-glykolpelargonat, Ester einer verzweigten Fettsäure mittlerer Kettenlänge mit gesättigten Fettalkoholen $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milch säureethylester, wachsartige Fettsäureester wie künstliches Entenbürzeldrüsenfett, Dibutylphthalat, Adipinsäurediisopropylester, letzterem verwandte Estergemische u.a.

Triglyceride wie Capryl/Caprinsäuretriglycerid, Triglyceridgemische mit Pflanzenfettsäuren der Kettenlänge $C_8$-$C_{12}$ oder anderen speziell ausgewählten natürlichen Fettsäuren, Partialglyceridgemische gesättigter oder ungesättigter evtl. auch hydroxylgruppenhaltige Fettsäuren, Mono, -Diglyceride der $C_8$/$C_{10}$-Fettsäuren und andere.

Fettalkohole wie Isotridecylalkohol, 2-Octyldodecanol, Cetylstearylalkohol, Oleylalkohol.

Fettsäuren wie z.B. Ölsäure.

Besonders gut geeignete spreitende Öle sind die folgenden:

Isopropylmyristat, Isopropylpalmitat, Capryl/Caprinsäureester von gesättigten Fettalkoholen der Kettenlänge $C_{12}$-$C_{18}$, wachsartige Fettsäureester wie künstliches Entenbürzeldrüsenfett.

Als Mittel für das erfindungsgemäße Verfahren kommen jedoch auch übliche Suspensions-und Emulsionskonzentrate in Frage, die vor Gebrauch mit Wasser auf die gewünschte Anwendungskonzentration verdünnt werden. Die Anwendungskonzentration des oder der Wirkstoffe liegt bei 0,1-10 %, bevorzugt bei 0,1-5 %. In den Formulierungen liegen die Wirkstoffe in Konzentrationen zwischen 5 und 80 % vor, bevorzugt zwischen 10 und 60 %.

Diese Formulierungen werden in bekannter Weise hergestellt, z.B. durch Vermischen des Wirkstoffs mit Streckmitteln, also z.B. flüssigen Lösungsmitteln, gegebenenfalls unter Verwendung von oberflächenaktiven Mitteln, also Emulgiermitteln und/oder Dispergiermitteln und/oder -schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummi arabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe enthalten sein.

Als Mittel für das erfindungsgemäße Verfahren kommen auch wasserhaltige Mikroemulsionen in Frage. Solche enthalten 0,1-10 % Wirkstoff, 1-30 % eines oberflächenaktiven Mittels, gegebenenfalls 20-70 % eines mit Wasser mischbaren Verdünnungsmittels sowie gegebenenfalls 0-5 % Hilfsmittel, wobei der Rest aus Wasser besteht.

Als oberflächenaktive Mittel seien genannt:

nicht-ionische, wasserlösliche Emulgatoren mit einem HLB (hydrophilic/lipophilicbalance-Wert) größer als 10, z.B. W® (Bayer AG), Alkylarylpolyglykolether, Emulgator NP 10® (Bayer AG), Alkylarylpolyglykolether; Renex 678® (Atlas Chemical Industries), Polyoxyethylenalkylarylether; Tween 40® -(Atlas), Polyoxyethylensorbitanmonopalmitat; Myri 53® (Atlas), Polyoxyethylenstearat; Atlas G 3707®, Polyoxyethylenlaurylether; Atlas G 3920®, Polyoxyethylen-oleylether; Atlas G 9046 T®, Polyoxyethylenmannitanmonolaurat; Emulgator 1371 B® (Bayer AG), Alkylpolyglykolether; Emulgator 1736® (Bayer AG), Alkylpolyglykolether (Oleylpolyglykolether); Emulgator OX® (Bayer AG), Alkylpolyglykolether (Dodecylpolyglykolether); Ninox BM-2® (Stepan Chemical Co.), ethoxyliertes Nonylphenol; Triton X-100® (Rohm und Haas Co.), Isooctylphenolpolyethoxyethanol; Cremophor EL®.

Als Lösungsmittel seien genannt: Methanol, Ethanol, Propanol, vorzugsweise Isopropanol, Dimethylsulfoxid, Dimethylformamid, Glycerin,Ethylen-glykolmonomethylether, Diethylenglykolmonomethylether, Methoxyethoxyethanol, Methyl-carbitol, Polyethylenglykole, Propylenglykole, Polypropylenglykole sowie Ketone wie Aceton und Methylethylketon.

Die Mittel werden hergestellt durch Lösen des oder der Wirkstoffe in einem Emulgator oder in seinem Emulgator-Lösungsmittelgemisch unter Erwärmung, falls notwendig, und durch Zugabe des erforderlichen Anteils Wasser unter Rühren. Eine besondere Homogenisierungsvorrichtung ist nicht erforderlich.

Aus den folgenden Beispielen geht die überlegene Wirkung der erfindungsgemäßen pour-on Formulierungen hervor.

So ist eine Formulierung mit Cyfluthrin einer Formulierung mit Permethrin als Wirkstoff hinsichtlich der Dauerwirkung, der Schnelligkeit des Wirkungseintritts und der Wirkhöhe überlegen. Eine Formulierung mit Fen fluthrin ist einer Formulierung mit Permethrin als Wirkstoff im Hinblick auf die Schnelligkeit des Wirkungseintritts und der Wirkhöhe überlegen.

Die erfindungsgemäßen pour-on Formulierungen können wegen der Schnelligkeit des Wirkungseintritts mit gutem Erfolg sowohl gegen nur kurzzeitig auf dem Wirtstier (Pferd, Rind, Schwein usw.) verweilende Parasiten wie stechende und leckende Fliegen also gegen sogenannte temporäre Parasiten, wie auch gegen permanente und periodische Parasiten, die dauernd oder in einzelnen Entwicklungsstadien auf den Wirtstieren parasitieren, eingesetzt werden.

Dieser Befund war überraschend und war nicht zu erwarten.

Aus den folgenden Versuchsbeispielen geht die gute Wirkung bei nicht direkt behandelten Herdentieren hervor.

Beispiel A

Die mit Parasiten infizierten Versuchsrinder wurden bei 23-25°C und 50-70 % r.F. in Ständen gehalten. Sie waren mit Hilfe eines Halsrahmens fixiert, um ein Lecken und Scheuern zu vermeiden.

Beiderseits eines mit der Pour-on Formulierung von Flumethrin in der Dosis von 1 mg/kg bzw. 0,3 mg/kg behandelten und wie oben beschrieben fixierten Rindes wurden je ein unbehandeltes ebenso fixiertes Rind aufgestallt.

Unbehandelte Kontrolltiere wurden räumlich getrennt so gehalten, daß sid keinerlei direkten Kontakt mit den Versuchsrindern hatten.

Als Maß für die Wirkung wurde die Zahl der zur Entwicklung gekommenen weiblichen Zecken von behandelten Tieren und von Kontrolltieren benutzt. Diese Wirkung wird in Prozent ausgedrückt.

# Tabelle A

Parasit: Boophilus microplus (OP-resistenter Biarra-Stamm)/Alle Entwicklungstadien (Rind)

| Wirkstoffdosis in mg/kg | Tage vor Behandl. -2 - +0 | Zahl der Zecken mit fertilen Gelegen | | | | | | | | Wirkung in % |
| --- | --- | --- | --- | --- | --- | --- | --- | --- | --- | --- |
| | | Tage nach Behandlung | | | | | | | | |
| | | 1-3 | 4-6 | 7-9 | 10-12 | 13-15 | 16-18 | 19-21 | $\Sigma$ +1-21 | |
| unbehandeltes Tier mit Kontakt | 246 | 7 | 0 | 0 | 0 | 0 | 4 | 1 | 12 | 98,8 |
| 1 | 241 | 0 | 0 | 0 | 0 | 0 | 0 | 0 | 0 | 100 |
| unbehandeltes Tier mit Kontakt | 189 | 14 | 0 | 0 | 0 | 0 | 0 | 0 | 14 | 97,7 |
| unbehandeltes Tier ohne Kontakt | 281 | 139 | 187 | 309 | 380 | 424 | 283 | 216 | 1938 | - |

0 214 477

## T a b e l l e   B

Parasit: Boophilus microplus (OP-resistenter Biarra-Stamm)/Alle Entwicklungstadien (Rind)

| Wirkstoffdosis in mg/kg | Zahl der Zecken mit fertilen Gelegen | | | | | | | | | Wirkung in % |
|---|---|---|---|---|---|---|---|---|---|---|
| | Tage vor Behandl. | Tage nach Behandlung | | | | | | | | |
| | -2 - +0 | 1-3 | 4-6 | 7-9 | 10-12 | 13-15 | 16-18 | 19-21 | $\sum$ +1-21 | |
| unbehandeltes Tier mit Kontakt | 204 | 12 | 0 | 0 | 1 | 3 | 9 | 2 | 27 | 97,3 |
| 0,3 | 211 | 0 | 0 | 1 | 1 | 0 | 2 | 1 | 5 | 99,7 |
| unbehandeltes Tier mit Kontakt | 186 | 0 | 3 | 4 | 0 | 0 | 2 | 10 | 19 | 97,9 |
| unbehandeltes Tier ohne Kontakt | 281 | 139 | 187 | 369 | 380 | 424 | 283 | 216 | 1938 | - |

| Flumethrin | 25,0 g |
|---|---|
| Calciumphenylsulfonat | 8,0 g |
| Alklarylpolyglykolether | 15,0 g |
| hochsiedende Erdölfraktion Solvesso 200® | ad 100 ml |

### Beispiel 2

Die so zusammengesetzte Formulierung wird vor der Anwendung im Verhältnis 1:10 mit Wasser verdünnt und im pour-on Verfahren ausgebracht.

| Cyfluthrin | 6,0 g |
|---|---|
| Butanol | 10,0 g |
| Nonylphenolethoxylat | 15,0 g |
| Natriumalkylbenzolfulsonat | 4,0 g |
| Wasser | ad 100 ml |

### Beispiel 3

Die so zusammengesetzte Formulierung wird vor der Anwendung im Verhältnis 1:6 mit Wasser weiter verdünnt und im pour-on Verfahren ausgebracht.

| Cyfluthrin | 40,0 g |
|---|---|
| Polyoxyethylen-sorbitan-fettsäureester | 5,0 g |
| ligninsulfonsaures Natrium | 7,0 g |
| kolloidale Kieselsäure | 32,0 g |
| Kaolin | 16,0 g |

### Beispiel 4

Die Komponenten werden gut vermischt und dann gemahlen. Vor Gebrauch wird die Formulierung in Wasser im Gewichts-Verhältnis 1:40 dispergiert und im pour-on Verfahren ausgebracht.

| | |
|---|---|
| Cyfluthrin | 1,0 g |
| hochsiedende Erdölfraktion Solvesso 200® | 6,0 g |
| Calciumphenylsulfonat | 6,0 g |
| Polyethylenglykolfettsäureether | 9,0 g |
| Wasser | ad 100 ml |

### Beispiel 5

Die so zusammengesetzte Formulierung wird ohne weitere Verdünnung im pour-on Verfahren ausgebracht.

| | |
|---|---|
| Cyfluthrin | 3,0 g |
| Nonylphenylpolyglykolether | 10,0 g |
| Dipropylenglykolmonomethylether | ad 100 ml |

### Beispiel 6

Die so zusammengesetzte Formulierung wird ohne weitere Verdünnung im pour-on Verfahren ausgebracht.

| | |
|---|---|
| Cypermethrin | 2,0 g |
| 2-Octyldodecanol | 20,0 g |
| Paraffinöl lt. Europ. Pharmakopoe | ad 100 ml |

### Beispiel 7

Die so zusammengesetzte Formulierung wird ohne weitere Verdünnung im pour-on Verfahren ausgebracht.

| | |
|---|---|
| Cyfluthrin | 0,5 g |
| Silikonöl | 30,0 g |
| Butylacetat | ad 100 ml |

### Beispiel 8

Die so zusammengesetzte Formulierung wird ohne weitere Verdünnung im pour-on Verfahren ausgebracht.

|  |  |
|---|---|
| Cyfluthrin | 0,5 g |
| Ethylstearat | 20,0 g |
| Isopropanol | ad 100 ml |

Die so zusammengesetzte Formulierung wird ohne weitere Verdünnung im pour-on Verfahren ausgebracht.

## Ansprüche

1. Verfahren zur Behandlung von Herdentieren und vergesellschaftet lebenden Tieren zur Bekämpfung von Ektoparasiten dadurch gekennzeichnet, daß man pro Herde ein oder mehrere Tiere im Aufgußverfahren mit einem Mittel behandelt das einen oder mehrere Wirkstoffe aus der Gruppe 2,2-Dimethyl-3-(2,2-dichlorvinyl)- cyclopropan-1-carbonsäure-α-cyano-(3-phenoxy-4-fluor)-benzylester (Cyfluthrin), 2,2-Dimethyl-3-[2-(4-chlorphenyl)-2-chlorvinyl)]-cyclopropan-1-carbonsäure-α-cyano-(3-phenoxy-4-fluor)-benzylester (Flumethrin), 2,2-Dimethyl-3-(2,2-dichlorvinyl)-cyclopropan-1-carbonsäure-α-cyano-3-phenoxy-benzylester (Cypermethrin), 2,2-Dimethyl-3-(2,2-dibromvinyl)-cyclopropan-1-carbonsäure-α-cyano-3-phenoxy-benzylester (Deltamethrin), 2,2-Dimethyl-3-(2,2-dichlorvinyl)-cyclopropan-1-carbonsäure-pentafluorbenzylester (Fenfluthrin), deren Enantiomere oder Diastereomere oder Gemische derselben neben hautverträglichen Verdünnungsmitteln sowie gegebenenfalls Emulgatoren und weiteren Hilfsstoffen enthält.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | JOURNAL OF ECONOMIC ENTOMOLOGY, Band 72, Nr. 4, 15. August 1979, Seiten 532-534, Entomological Society of America, College Park, Maryland, US; T.L. HARVEY et al.: "Treatment of one beef animal per herd with permethrin for horn fly control" * Seite 532, linke Spalte, Absätze 1,2; Seite 533, rechte Spalte, letzter Absatz * | 1 | A 01 N 25/00 A 01 N 53/00 |
| | --- | | |
| A | EP-A-0 120 286 (THE WELLCOME FOUNDATION) * Anspruch 11 * | 1 | |
| | --- | | |
| A | EP-A-0 045 424 (BAYER) | 1 | |
| | --- | | RECHERCHIERTE SACHGEBIETE (Int. Cl.4) |
| A | EP-A-0 069 269 (BAYER) | 1 | |
| | --- | | A 01 N |
| A | R. WEGLER: "Chemie der Pflanzenschutz- und Schädlingsbekämpfungsmittel", Band 7, 1981, Seiten 10-16, Springer-Verlag, Berlin, DE * Seite 13; Seite 15, letzer Absatz - Seite 16 * | 1 | |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 01-12-1986 | DECORTE D. |